# EUROPÄISCHE PATENTSCHRIFT

(11) **EP 1 330 255 B1**
(45) Veröffentlichungstag und Bekanntmachung des Hinweises auf die Patenterteilung: **15.06.2005**
(21) Anmeldenummer: 01992579.1
(22) Anmeldetag: 12.10.2001
(51) Int. Cl.: A61K 35/78, A61P 13/10

(54) **ZUBEREITUNG VON CIMICIFUGA RACEMOSA ZUR BEHANDLUNG DER INKONTINENZ**
PREPARATION OF CIMICIFUGA RACEMOSA FOR TREATING INCONTINENCE
PREPARATION DE CIMICIFUGA RACEMOSA POUR TRAITER L'INCONTINENCE

(30) Priorität: 03.11.2000 DE 10054641
(43) Veröffentlichungstag der Anmeldung: 30.07.2003
(73) Patentinhaber: Schaper & Brümmer GmbH & Co. KG, 38259 Salzgitter (DE)
(72) Erfinder: GESSLER, Andrea, C., 37130 Gleichen-Reinhausen (DE); NISSLEIN, Thomas, 37154 Northeim (DE)
(74) Vertreter: Lins, Edgar, Dipl.-Phys. Dr.jur.
(86) Internationale Anmeldenummer: PCT/DE2001/003926
(87) Internationale Veröffentlichungsnummer: WO 2002/036140

(56) Entgegenhaltungen:
- WO-A-99/47149
- LISKE, E: "Therapeutic Efficacy and Safety of Cimicifuga racemosa for Gynecologic Disorders" ADVANCES IN THERAPY, Bd. 15, Nr. 1, 1998, Seiten 45-53, XP001068814
- KRUSE, SVEN O. ET AL: "Fukiic and piscidic acid esters from the rhizome of Cimicifuga racemosa and the in vitro estrogenic activity of fukinolic acid" PLANTA MED. (1999), 65(8), 763-764 , XP001069505
- DATABASE BIOSIS [Online] BIOSCIENCES INFORMATION SERVICE, PHILADELPHIA, PA, US; 1996 EINER-JENSEN N ET AL: "Cimicifuga and Melbrosia lack oestrogenic effects in mice and rats." Database accession no. PREV199799295698 XP002196936 & MATURITAS, Bd. 25, Nr. 2, 1996, Seiten 149-153, ISSN: 0378-5122
- "The Merck Manual of Diagnosis and Therapy" 1999 , MERCK RESEARCH LABORATORIES XP002196935 Seite 1942 -Seite 1943

## Beschreibung

Die Erfindung betrifft eine Verwendung einer Zubereitung von Cimicifuga racemosa (Traubensilberkerze) insbesondere eines Extraktes und insbesondere aus dem Wurzelstock (Rhizoma cimicifugae racemosae).

Extrakte von Cimicifuga racemosa werden in der Gynäkologie für die Behandlung von Wechseljahresbeschwerden, wie Hitzewallungen, Schweißausbrüchen, Schlafstörungen, Nervosität und depressiven Verstimmungszuständen, eingesetzt. Der Extrakt gilt als pflanzliche Alternative zur Oestrogenersatztherapie. In der allopathischen Tiermedizin spielen die Extrakte von Cimicifuga racemosa keine bedeutende Rolle.

Überraschender Weise ist gefunden worden, dass Zubereitungen von Cimicifuga racemosa beim Vorliegen einer Harninkontinenz therapeutisch wirksam sind.

Es ist bekannt, dass Hündinnen nach der Kastration, also nach dem Beseitigen der Ovarien, evtl. einschließlich der Gebärmutter, durchschnittlich nach etwa 2,7 Jahren nach der Ovario-Hysterektomie zu einem Prozentsatz von etwa 20 % an Harninkontinenz als Folgesymptom des Eingriffes leiden. Das Krankheitsbild wird heute vor allem durch Sympathomimetika behandelt, die jedoch bei nicht optimal eingestellten Tieren massive Nebenwirkungen verursachen.

Es ist früher vermutet worden, dass die Harninkontinenz auf die sich durch den Eingriff einstellende Oestrogendefizienz zurückzuführen ist. Trotz Zweifel an diesem Kausalzusammenhang, die durch das Auftreten erst durchschnittlich 2,7 Jahre nach der Ovariektomie geweckt werden, wurde versucht, das Krankheitsbild durch Oestrogensubstitution zu behandeln. Diese Zweifel in Verbindung mit den unter der Therapie auftretenden Nebenwirkungen haben dazu geführt, dass zunehmend die Behandlung mit Sympathomimetika in Betracht gezogen worden ist.

Bei über 60-jährigen Frauen mit einer basalen Inkontinenzinzidenz von 10 bis 33 % steigert die Hysterektomie, also die Entfernung der Gebärmutter ohne Eierstöcke, d.h. auch ohne zusätzliche Einflussnahme auf den Hormonstatus, die Wahrscheinlichkeit, Harninkontinenz zu entwickeln, um 60 %. Als Ursache für diesen Zusammenhang werden operationsbedingte Läsionen an Nerven und Bänder-Bindegewebs-Strukturen des Beckenbereichs diskutiert (Lancet 2000; 356(9229): 535-539). Gleichartige Läsionen sind auch bei der in der Veterinärmedizin typischen Operationsweise die Regel. Die erfindungsgemäße Wirksamkeit der Zubereitung von Cimicifuga racemosa zur Behandlung von Harninkontinenz beruht somit nicht auf den bisher Cimicifuga racemosa zugeschriebenen Eigenschaften als Oestrogenersatz bzw. als endokrin aktives Phytotherapeutikum, sondern vielmehr auf Wirkungen außerhalb der bekannten Eigenschaften

Obwohl bezüglich der Übertragbarkeit von veterinärmedizinischen Kausal- und Therapiebefunden auf humanmedizinische Symptomenkomplexe Vorsicht geboten ist, legen die gefundenen Ergebnisse auch eine Wirksamkeit für die Behandlung von Harninkontinenz bei ovariektomierten oder peri- bzw. postmenopausalen Frauen nahe. Das Auftreten der Harninkontinenz bei postmenopausalen Frauen liegt altersabhängig zwischen 20 und 50 %. In der Perimenopause wird keine Zunahme beobachtet. Eine frühe Oestrogensubstitution ist mit einer nur kurzzeitigen, geringen Symptomverbesserung, langfristig jedoch mit einer Erhöhung des Inkontinenzrisikos bei über 60-jährigen Frauen verbunden, wie in einer Studie (D. H. Thom, J. S. Brown in J Am Geriatr Soc 1998; 46(11): 1411-1417) veröffentlicht worden ist.

Der bisherige Wissenstand konnte daher die erfindungsgemäße Verwendung von Cimicifuga racemosa zur Behandlung der Harninkontinenz nicht nahelegen und kann deren beobachtete Erfolge nicht erklären.

Als wirksame Tagesdosis für die erfindungsgemäße Verwendung ist ein Drogengehalt zwischen 0,1 und 10 mg/kg Körpergewicht ermittelt worden. Es ist daher zweckmäßig, einen Extrakt in Tablettenform mit einem Drogengehalt zwischen 5 und 200 mg, vorzugsweise zwischen 10 und 50 mg pro Tablette zu formulieren.

Erfindungsgemäß wird vorzugsweise ein Extrakt verwendet, der mit einem alkoholischen Extraktionsmittel, insbesondere mit Ethanol, Isopropanol und Methanol, hergestellt worden ist.

Die Verabreichung kann in allen pharmazeutisch gebräuchlichen Darreichungsformen erfolgen, z.B. als Granulat, Kapseln, Suppositorien, Tabletten, Lösungen, Teezubereitungen oder transdermal mit Pflaster o.dgl.

Es kommt auch eine Beimischung in einem Lebensmittel oder Tierfutter in Betracht.

Die Zubereitung muss nicht unbedingt als Extrakt verabreicht werden. Beispielsweise ist auch das Vermahlen der Pflanze oder von Pflanzenteilen zur unmittelbaren oder mittelbaren - beispielsweise als Tee - Einnahme möglich.

Die Therapie mit der erfindungsgemäßen Zubereitung aus Cimicifuga racemosa kommt für weibliche Säugetiere nach einer Ovario-Hysterektomie und für Frauen nach einer Hysterektomie oder nach der Menopause in Betracht.

### Beispiel:

Bei ovariektomierten Hündinnen mit der Symptomatik der Harninkontinenz ist eine Behandlung mit einem Extrakt von Rhizoma cimicifugae racemosae in Tablettenform ("Remifemin"-Tabletten der Schaper & Brümmer GmbH & Co. KG) vorgenommen worden. Als wirksame Tagesdosis wurde eine halbe bis eine Tablette pro 10 kg Körpergewicht angewendet. In den vorgenommenen vierzehn Fällen kam es zur Remission der Inkontinenz. Das subjektive Empfinden der Patientenbesitzer zum Therapieerfolg und zur Verträglichkeit des Medikamentes waren durchweg positiv. In allen Fällen wurde um Fortsetzung der Therapie gebeten.

In allen Fällen kam es zu einer deutlichen Besserung des Zustandes bis hin zur völligen Beschwerdefreiheit.

Die verwendeten Remifemin®-Tabletten enthalten einen Extrakt aus 20 mg Droge pro Tablette. Als Auszugsmittel ist Isopropanol 40 vol.% verwendet worden.

## Patentansprüche

1. Verwendung einer Zubereitung aus Cimicifuga racemosa zur Herstellung eines Arzneimittels zur Behandlung der Harninkontinenz bei weiblichen Säugern nach einer Ovario-Hysterektomie oder nach der Menopause.

2. Verwendung eines Extraktes von Cimicifuga racemosa zur Herstellung eines Arzneimittels nach Anspruch 1.

3. Verwendung eines mit einem alkoholischen Extraktionsmittel hergestellten Extraktes nach Anspruch 2.

4. Verwendung eines Extraktes nach Anspruch 2 oder 3 in Tablettenform mit einem Drogengehalt zwischen 5 und 200 mg pro Tablette.

5. Verwendung einer Zubereitung nach einem der Ansprüche 1 bis 4 als Beimischung in einer Tierfuttermischung.

## Claims

1. Use of a preparation of Cimicifuga racemosa for producing a medicinal product for treatment of urinary incontinence in female mammals following an ovariohysterectomy or after the menopause.

2. Use of an extract of Cimicifuga racemosa for producing a medicinal product according to Claim 1.

3. Use of an extract according to Claim 2 which has been produced using an alcoholic extracting agent.

4. Use of an extract according to Claim 2 or 3 in the form of tablets having a drug content of between 5 and 200 mg per tablet.

5. Use of a preparation according to any one of Claims 1 to 4 as an additive in an animal feed mix.

## Revendications

1. Utilisation d'une préparation à base de Cimicifuga racemosa pour la préparation d'un médicament pour le traitement de l'incontinence urinaire chez les mammifères femelles, après une ovario-hystérectomie ou après la ménopause.

2. Utilisation d'un extrait de Cimicifuga racemosa pour la préparation d'un médicament selon la revendication 1.

3. Utilisation d'un extrait selon la revendication 2 préparé à partir d'un produit d'extraction alcoolique.

4. Utilisation d'un extrait selon la revendication 2 ou 3, sous la forme de tablettes avec une teneur en principe actif comprise entre 5 et 200 mg par tablette.

5. Utilisation d'une préparation selon l'une des revendications 1 à 4, en tant qu'additif de mélange dans un mélange d'aliment pour animaux.
